# EUROPÄISCHE PATENTSCHRIFT

(11) **EP 0 957 081 B1**
(45) Veröffentlichungstag und Bekanntmachung des Hinweises auf die Patenterteilung: **08.01.2003**
(21) Anmeldenummer: 99108789.1
(22) Anmeldetag: 03.05.1999
(51) Int. Cl.: C07C 227/12, C07C 235/00

(54) **Verfahren zur Herstellung von N-Acylaminosäuren**
Process for preparing N-acyl aminoacids
Procédé de préparation d'amino-acides N-acylés

(30) Priorität: 13.05.1998 DE 19821380
(43) Veröffentlichungstag der Anmeldung: 17.11.1999
(73) Patentinhaber: Degussa AG, 40474 Düsseldorf (DE)
(72) Erfinder: Beller, Matthias Prof., 18119 Rostock (DE); Eckert, Markus, 80505 München (DE); Moradi, Wahed, 80939 München (DE)

(56) Entgegenhaltungen:
- DE-A- 3 242 374
- DE-C- 19 629 717

## Beschreibung

Die Erfindung betrifft ein Verfahren zur Herstellung von N-Acylaminosäuren der allgemeinen Formel I 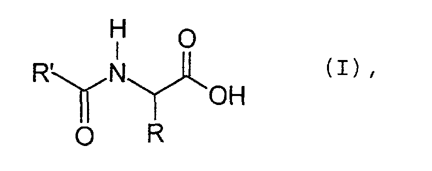 worin
R Wasserstoff, eine Carboxylgruppe, eine (C₁-C₁₂)-Alkylgruppe, die gesättigt, geradkettig, verzweigt oder zyklisch vorliegen kann, einen (C₂-C₁₂)-Alkenylrest, der ein- oder mehrfach ungesättigt, geradkettig, verzweigt oder zyklisch vorliegen kann, und eine (C₁-C₈)-Acyloxygruppe sowie einen (C₅-C₁₈)-Arylrest,
R' Wasserstoff, einen gesättigten, geradkettigen, verzweigten oder zyklischen (C₁-C₂₆)-Alkyl, einen ein- oder mehrfach ungesättigten, geradkettigen, verzweigten oder zyklischen (C₂-C₂₄)-Alkenylrest, einen (C₆-C₁₈)-Alykl-(C₅-C₁₈)-Arylrest oder einen ggf. mehrfach ungesättigten (C₂-C₁₀)-Alkenyl-(C₅-C₁₈)-Arylrest, bedeutet.

N-Acylaminosäuren sind wichtige Ausgangsprodukte bei der Peptidsynthese sowie Zwischenprodukte zur Herstellung von bioaktiven Wirkstoffen. Darüber hinaus sind sie als Detergenzien, Bohrmittel-Zusatzstoffe und als Nahrungsmittelzusätze bedeutsam.

Es ist bekannt N-Acylaminosäuren durch Acylierung entsprechender Aminosäuren unter Anfall von Salznebenprodukten herzustellen. Im Falle nicht-natürlicher Aminosäuren muß die entsprechende Aminosäure vorab häufig in mehreren Stufen hergestellt werden. Ein Ein-Stufen-Verfahren, das die genannten Nachteile vermeidet, ist die Amidocarbonylierung von Aldehyden und Amiden, die im folgenden Schema dargestellt ist.

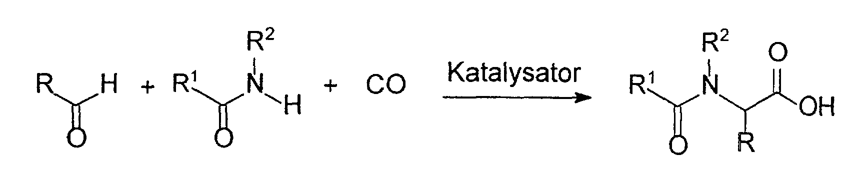

Die Amidocarbonylierung wurde zuerst von Wakamatsu et al., (Chemical Communications 1971, Seite 1540 und in DE-A2-21 15 985) beschrieben. Die Carbonylierung wird in Gegenwart von Wasserstoffgas mit einem Mol-Verhältnis CO : H₂ = 3 : 1 durchgeführt. Als Katalysator dient der Cobaltcarbonyl-Komplex Co₂(CO)₈, der in einer Konzentration von 30 mmol Co-Metall je Liter Reaktionsgemisch eingesetzt wurde.

Ein weiteres cobalt-katalysiertes Verfahren, basierend auf der Amidocarbonylierung, wird in GB 2 252 770 beschrieben. Dort gelingt die Synthese von N-Acylaminosäuren durch Umsetzung eines Carbonsäureamids mit einem Aldehyd und CO in Gegenwart eines Metallkatalysators und einer Säure als Cokatalysator.

EP-B-0 338 330 beschreibt ein Verfahren zur Herstellung von N-Acylglycinderivaten durch Verwendung eines Katalysatorsystems, bestehend aus einer Palladiumverbindung und einem ionischen Halogenid. In DE 195 45 641 und der DE 196 29 717 wird ein Verfahren zur Darstellung von N-Acylglycinderivaten aus einem Carbonsäureamid und einem Aldehyd unter Palladium Katalyse beschrieben. Als Cokatalysatoren werden hier ionische Halogenide und zusätzlich Säure eingesetzt.

Die bekannten Verfahren gehen bei der Amidocarbonylierung von Carbonsäureamiden aus. In vielen Fällen ist jedoch ein solches Carbonsäureamid großtechnisch nicht in ausreichendem Maße kostengünstig zu erhalten, so daß einer technischen Realisierung eines entsprechenden Verfahrens die hohen variablen Kosten der Carbonsäureamide als Startmaterialien entgegenstehen.

Aufgabe der vorliegenden Erfindung war es deshalb, ein verbessertes Verfahren zur Herstellung von N-Acylaminosäuren durch Amidocarbonylierung anzugeben, das es gestattet, die genannte Reaktion in kostengünstigerer Weise durchführen zu können.

Gegenstand der Erfindung ist ein Verfahren zur Herstellung von N-Acylaminosäuren der allgemeinen Formel I 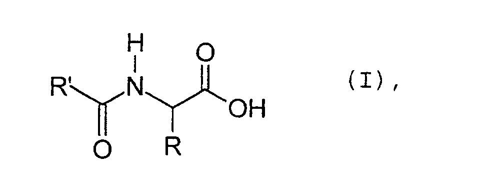 worin
R Wasserstoff, eine Carboxylgruppe, eine (C₁-C₁₂)-Alkylgruppe, die gesättigt, geradkettig, verzweigt oder zyklisch vorliegen kann, einen (C₂-C₁₂)-Alkenylrest, der ein- oder mehrfach ungesättigt, geradkettig, verzweigt oder zyklisch vorliegen kann, und eine (C₁-C₈)-Acyloxygruppe sowie einen (C₅-C₁₈)-Arylrest,
R' Wasserstoff, einen gesättigten, geradkettigen, verzweigten oder zyklischen (C₁-C₂₆)-Alkyl, einen ein- oder mehrfach ungesättigten, geradkettigen, verzweigten oder zyklischen (C₂-C₂₄)-Alkenylrest, einen (C₆-C₁₈)-Alykl- (C₆-C₁₈)-Arylrest oder einen ggf. mehrfach ungesättigten (C₂-C₁₀)-Alkenyl-(C₅-C₁₈)-Arylrest, bedeutet,
   welches dadurch gekennzeichnet ist, daß man ein Nitril der allgemeinen Formel II

   R'CN (II),

   in dem R' die oben angegebene Bedeutung besitzt, mit einem Aldehyd der allgemeinen Formel III

   R―CHO (III),

   in dem R die oben angegebene Bedeutung besitzt, in Gegenwart von einer Säure, Kohlenmonoxid und einem Metallkatalysator umsetzt. Dadurch gelangt man in äußerst kostengünstiger Weise zu den gewünschten Verbindungen der allgemeinen Formel I. Nitrile bilden in fast allen Fällen die Vorstufe zu den entsprechenden Carbonsäureamiden, wodurch sich deren geringerer Preis erklären läßt.

Erfindungsgemäß können beliebige Nitrile verwendet werden. Beispiele für geeignete Nitrile sind Acetonitril, Benzonitril, substituierte Benzonitrile, Benzylcyanid, Acrylnitril, Malondinitril, Adiponitril, Butylcyanid, Allylcyanid, Mandelsäurenitril und Fettsäurenitrile.

Für das erfindungsgemäße Verfahren können beliebige Aldehyde eingesetzt werden, z.B. Formaldehyd, Acetaldehyd, Propionaldehyd, Butyraldehyd, Valeraldehyd, 2-Ethylhexanal, Isobutyraldehyd, Furfural, Crotonaldehyd, Acrolein, Benzaldehyd, substituierte Benzaldehyde, Phenylacetaldehyd, 2,4-Dihydroxyphenylacetaldehyd, Glyoxalsäure und α-Acetoxypropionaldehyd. Es können auch Dialdehydverbindungen eingesetzt werden. Ebenfalls geeignet sind Substanzen, die unter den genannten Reaktionsbedingungen einen Aldehyd bilden können, z. B. Aldehydoligomere, wie Paraformaldehyd, Acetale, Allylalkohole und Epoxide.

Die Aldehyde können in die Reaktion in Form ihrer Trimeren oder Oligomeren eingesetzt werden.

Als Säure können im Prinzip alle dem Fachmann für die Nitrilhydrolyse in Frage kommenden Verbindungen verwendet werden. Bevorzugt ist der Einsatz von Säuren mit einem pka-Wert von < 4. Bevorzugt kann man Schwefelsäure oder ein Hydrogenhalogenid, wie Hydrogenchlorid oder Hydrogenbromid, zu dieser Reaktion einsetzen. Es können auch Säuregemische solch starker Säuren eingesetzt werden. Als besonders bevorzugte Variante kann eine Mischung einer starken Säure wie z.B. Schwefelsäure oder Hydrogenbromid in Gegenwart von Ameisensäure eingesetzt werden. Die Ameisensäure kann in 1-100 Äquivalenten bezogen auf das Nitril benutzt werden.

Als aktive Metallkatalysatoren kommen für die betrachtete Reaktion im Prinzip alle dem Fachmann bekannten Katalysatoren in Frage. Bevorzugt sind Metallkatalysatoren aus Palladium-(0)- bzw. Cobalt-(0)-Verbindungen.

Als Cobaltkatalysatoren bzw. -präkatalysatoren werden vorzugsweise Cobaltcarbonyle, z. B. festes Co₂(CO)₈, eingesetzt. Das Cobaltcarbonyl kann jedoch auch aus bekannten Cobalt(II)- und Cobalt(III)-verbindungen, wie z. B. Cobalt(II)acetat, Cobalt(II)chlorid, Cobalt(II)bromid in Gegenwart von CO, ggf. unter Zusatz von H₂, in situ gebildet werden.

Als Palladiumkatalysatoren bzw. -präkatalysatoren können beliebige Palladium(II)-verbindungen, Palladium(0)-verbindungen und Palladium auf Trägermaterialien, wie beispielsweise auf Palladium auf Aktivkohle, eingesetzt werden. Beispiele für Palladium-II-verbindungen sind Palladiumacetat, Palladiumhalogenide, Palladiumnitrile, Palladiumnitrate, Palladiumcarbonate, Palladiumketonate, Palladiumacetylacetonate sowie Allylpalladiumkomplexe. Besonders bevorzugte Vertreter sind PdBr₂, PdCL₂, Li₂PdBr₄, Li₂PdCl₄ und Pd(OAc)₂. Beispiele für Palladium-0-verbindungen sind Palladiumphosphinkomplexe und Palladiumolefinkomplexe. Besonders bevorzugte Vertreter sind Palladium(dba)-komplexe (dba = dibenzylidenaceton) und Pd(PPh₃)₄.

Beim Einsatz von Palladiumphosphinkomplexen haben sich zudem besonders Bisphosphin-Palladium(II)-Verbindungen bewährt. Die Komplexe können als solche eingesetzt werden oder in der Reaktionsmischung aus einer Palladium-IIverbindung, wie z. B. PdBr₂, PdCl₂ oder Palladium-II-acetat unter Zusatz von Phosphinen, wie z. B. Triphenylphosphin, Tritolylphosphin, Bis-(diphenylphosphino)-ethan, 1,4-Bis-(diphenylphosphino)-butan oder 1,3-Bis-(diphenylphosphino)-propan, erzeugt werden.

Von den genannten Palladiumphosphinkomplexen sind Bistriphenylphosphin-Palladium (II) bromid-PdBr₂ (PPh₃)₂- und das entsprechende Chlorid besonders bevorzugt. Diese Komplexe können als solche eingesetzt werden oder in der Reaktionsmischung aus Palladium(II)bromid bzw. -chlorid und Triphenylphosphin erzeugt werden.

Für das erfindungsmäßige Verfahren hat sich gezeigt, daß eine Menge von 0.0001 bis 5 mol% Palladiumverbindung (berechnet auf Palladiummetall), bevorzugt von 0.001 - 4 mol% und besonders bevorzugt von 0.01 - 2 mol% bezogen auf das Nitril, ausreichend ist.

Als Halogenidsalz können z. B. Phosphoniumbromide und Phosphoniumiodide, z. B. Tetrabutylphosphoniumbromid bzw. Tetrabutylphosphoniumiodid, sowie Ammonium-, Lithium-, Natrium-, Kaliumchlorid, -bromid und -iodid verwendet werden. Bevorzugte Halogenide sind die Chloride und Bromide. Vorzugsweise wird das ionische Halogenid in einer Menge von 1 bis 100 mol%, insbesondere von 2 - 40 mol% und ganz besonders von 5 - 30 mol%, bezogen auf das Nitril, eingesetzt.

Als Lösungsmittel können prinzipiell alle dem Fachmann geläufigen organischen Verbindungen eingesetzt werden. Bevorzugt sind dipolar aprotische Verbindungen einsetzbar. Beispiele dafür sind Dioxan, Tetrahydrofuran, N-Methylpyrrolidon, Ethylenglykoldimethylether, Essigsäureethylester, Essigsäure, Acetonitril, Benzonitril, tert.Butylmethylether, Dibutylether, Sulfolan, N,N-Dimethylacetamid oder Gemische davon. Die Lösungsmittel können in reiner Form oder produktenthaltend bzw. mit Produkt gesättigt eingesetzt werden. Bevorzugt sind N-Methylpyrrolidon, Dimethylformamid und Acetontril als Lösungsmittel.

Die Reaktion kann bei Drücken von 1 bis 250 bar, vorzugsweise von 10 bis 150 bar, und bei Temperaturen von 0 - 200 °C, vorzugsweise von 50 - 150 °C, durchgeführt werden.

Das erfindungsgemäße Verfahren kann als "Eintopfverfahren" oder bevorzugt in zwei Stufen durchgeführt werden. Beim 2-Stufen-Prozeß wird das Nitril zunächst zu einer Mischung aus Wasser und einer Säure, z. B. konz. Schwefelsäure, getropft. Nach Zugabe von Lösungsmittel, Aldehyd, Cobaltoder Palladiumkatalysator und ionischem Halogenid wird das Gemisch mit Kohlenmonoxid umgesetzt. Dabei werden für den Gesamtprozeß isolierte Ausbeuten von bis zu 99 % zur N-Acylaminosäure erreicht.

Falls gewünscht, kann das Verfahren auch in einer Stufe durchgeführt werden. Dazu löst man beispielsweise den Aldehyd, die Palladiumverbindung und das Halogenid in dem Nitril und tropft dieses Gemisch zu der Säure/Wasser-Mischung und setzt es in Gegenwart von Kohlenmonoxid zum Endprodukt um.

Des weiteren ist es möglich, durch eine chirale Modifizierung des Metallkatalysators einen sehr einfachen Zugang zu enantiomer angereicherten N-Acylaminosäuren zu erhalten.

Unter einem (C₅-C₁₈)-Arylrest versteht man z. B. einen ggf. substituierten Phenyl-, Naphthyl-, Anthryl-, Phenanthryl-, Biphenylrest oder ein fünf-, sechs- oder siebengliedrigen Heteroaromat mit ggf. Stickstoff-, Sauerstoff- oder Schwefelatomen im Ring, wobei diese Reste mit Fluor, Chlor, Brom, Iod, OH, NO₂, CN, CO₂H, CHO, SO₃R'', SO₂R'', SOR'', NHCOR'', COR'', NHCHO, COAr, CO₂Ar, CF₃, CONH₂, CHCHCO₂R'', SiR'', POAr₂, POR'' substituiert sein können.

Unter einem (C₁-C₁₂)-Alkylrest versteht man einen Alkylrest mit einem bis zwölf C-Atomen, welcher alle Bindungsisomeren, die für einen derarteigen Rest vorstellbar sind, mit umfaßt. Dieser kann auch als Carbocyclus vorliegen. Entsprechendes gilt für den (C₂-C₂₆)-Alkylrest. Unter einem (C₂-C₁₂)-Alkenylrest versteht man einen Alkenylrest mit zwei bis zwölf C-Atomen, welcher alle Bindungsisomeren, die für einen derartigen Rest vorstellbar sind, mit umfaßt. Dieser kann auch als Carbocyclus vorliegen. Entsprechendes gilt für den (C₂-C₂₄)-Alkenylrest. Unter einem (C₁-C₈)-Acyloxyrest vesteht man eine linear oder verzweigten Alkylgruppe mit einem bis acht C-Atomen samt aller bei diesem Rest vorstellbaren Bindungsisomeren, welche über eine Carbonyloxyfunktion an das Molekül gebunden ist.

Die im Rest R und R' vorkommenden Alkyl- und Alkenylgruppen können dabei mit Fluor, Chlor, Brom, Iod, OH, NO₂, CN, CO₂H, CHO, SO₃R'', SO₂R'', SOR'', NHCOR'', COR'', NHCHO, COAr, CO₂Ar, CF₃, CONH₂, CHCHCO₂R'', SiR'', POAr₂, POR'' substituiert sein.

Die Abkürzung Ar steht für einen (C₅-C₁₈)-Arylrest.

R'' bedeutet einen (C₁-C₁₂)-Alkylrest der gesättigt, gradkettig, verzweigt oder zyklisch vorliegen kann, einen (C₂-C₁₂)-Alkenylrest, der ein- oder mehrfach ungesättigt, gradkettig, verzweigt oder zyklisch vorliegen kann.

Bis dato wurde eine erfindungsgemäße in situ Präparierung des Carbonsäureamids aus dem Carbonsäurenitril für Amidocarbonylierungen noch nie vorgeschlagen. Das ist u. a. darauf zurückzuführen, daß eine selektive Präparierung von Carbonsäureamiden aus Nitrilen unter Amidocarbonylierungsbedingungen prinzipiell problematisch ist. Aufgrund der bei der Amidocarbonylierung im ersten Schritt freigesetzten Menge an Wasser, war zu erwarten, daß bei hoher Säurekonzentration das noch nicht umgesetzte Carbonsäureamid zur Carbonsäure hydrolysiert wird und es somit zu einer substantiellen Ausbeuteminderung kommt. Darüber hinaus sollte die zur schnellen Nitrilhydrolyse notwendige Menge an Säure zur Erhöhung der Nebenreaktionen zwischen Carbonsäureamiden und Aldehyden führen und den Metallkatalysator in seiner Aktivität und Lebensdauer beeinträchtigen.

Die folgenden Beispiele sollen die Erfindung erläutern, sie jedoch keinesfalls einschränken.

### Beispiele

### Beispiel 1:

1.1 g Acetonitril werden unter Rühren zu einer Mischung aus 2.5 g Schwefelsäure und 0.5 g Wasser getropft und anschließend in 25 ml N-Methylpyrrolidon mit 2.8 g Cyclohexylcarbaldehyd, 0.05 g Bis(triphenylphosphin)-palladium(II)bromid und 0.76 g Lithiumbromid bei 60 bar Kohlenmonoxiddruck und 120 °C in einem 300-ml-Autoklaven umgesetzt. Nach einer Reaktionszeit von 12 Stunden wird das Gemisch mittels Hochdruckflüssigkeitschromatographie (HPLC) analysiert. Man findet 4.6 g N-Acetylcyclohexylglycin; dies entspricht einer Ausbeute von 92 %.

### Beispiel 2:

2.6 g Benzonitril werden unter Rühren zu einer Mischung aus 2.5 g Schwefelsäure und 0.5 g Wasser getropft und anschließend in 25 ml N-Methylpyrrolidon mit 2.8 g Cyclohexylcarbaldehyd, 0.266 g Palladium auf Aktivkohle (10%ig) und 0.76 g Lithiumbromid bei 60 bar Kohlenmonoxiddruck und 120 °C in einem 300-ml-Autoklaven umgesetzt. Nach einer Reaktionszeit von 12 Stunden wird das Gemisch mittels Hochdruckflüssigkeitschromatographie (HPLC) analysiert. Man findet 4.58 g N-Benzoylcyclohexylglycin; dies entspricht einer Ausbeute von 70 %.

### Beispiel 3:

Im Autoklaven wird eine Lösung von 2.8 g Cyclohexylcarbaldehyd, 0.05 g Bis(triphenylphosphin)-palladium(II)bromid und 1.5 g Tetrabutylammoniumbromid in 25 ml Acetonitril unter Rühren zu einer Mischung aus 2.5 g Schwefelsäure und 0.5 g Wasser getropft und bei 60 bar Kohlenmonoxiddruck und 120 °C umgesetzt. Nach einer Reaktionszeit von 12 Stunden wird das Gemisch mittels Hochdruckflüssigkeitschromatographie (HPLC) analysiert. Man findet 1.6 g N-Acetylcyclohexylglycin; dies entspricht einer Ausbeute von 32 %.

### Beispiele 4 - 9:

Analog zu Beispiel 1 wurden folgende N-Acylaminosäuren hergestellt (Tabelle 1) :

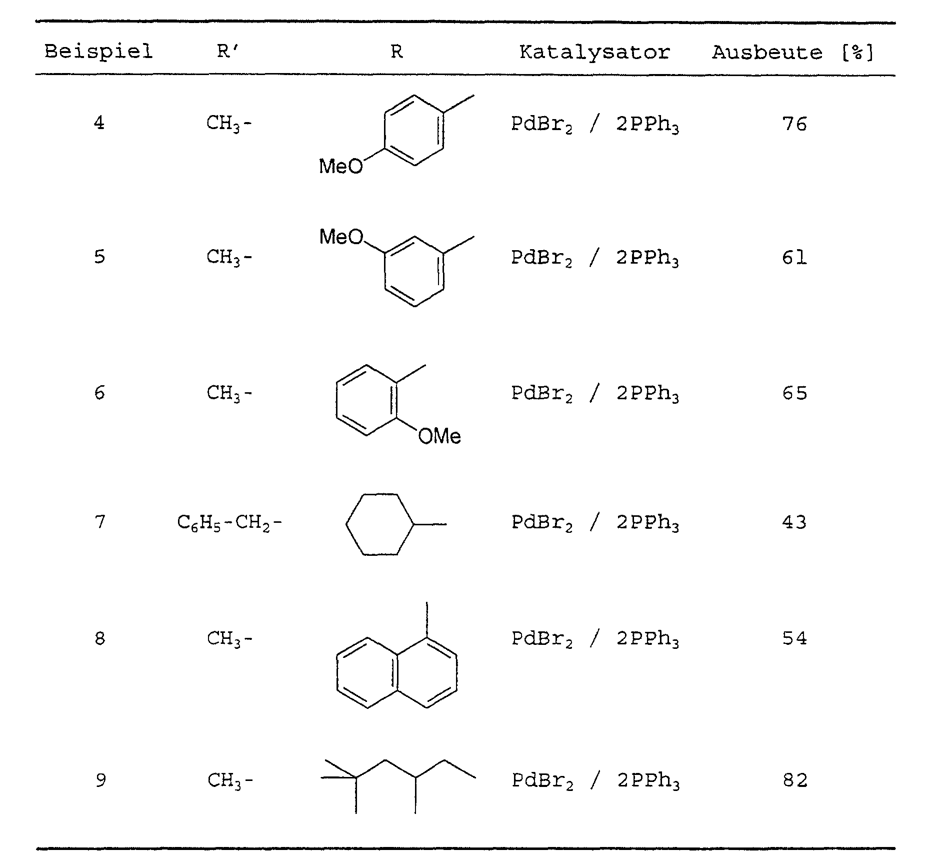

### Beispiel 10:

25 g Acetonitril werden in 1,2 g Ameisensäure und 0,8 g Hydrogenbromid (in Eisessig) zwei Stunden bei Raumtemperatur gerührt und anschließend mit 2,8 g Cyclohexylcarbaldehyd, 0,05 g Bis(triphenylphosphan)-palladium(II)dibromid bei 60 bar Kohlenmonoxiddruck und 100°C in einem 300 ml Autoklaven umgesetzt. Nach 12 Stunden Reaktionszeit wird das Gemisch mittels Hochdruckflüssigkeitschromatographie (HPLC) analysiert. Man erhält 1,5 g N-Acetylcyclohexylglycin, was einer Ausbeute von 30% entspricht.

### Beispiel 11:

25 g Acetonitril werden in 1,2 g Ameisensäure und 1,5 g Tetrabutylammoniumbromid, 0,5 g Schwefelsäure, 2,8 g Cyclohexylcarbaldehyd und 0,05 g Bis(triphenylphosphan)-palladium(II)dibromid zwei Stunden bei Raumtemperatur gerührt und anschließend bei 60 bar Kohlenmonoxiddruck und 100°C in einem 300 ml Autoklaven umgesetzt. Nach 12 Stunden Reaktionszeit wird das Gemisch mittels Hochdruckflüssigkeitschromatographie (HPLC) analysiert. Man erhält 1,1 g N-Acetylcyclohexylglycin, was einer Ausbeute von 22% entspricht.

## Patentansprüche

1. Verfahren zur Herstellung von N-Acylaminosäuren der allgemeinen Formel I 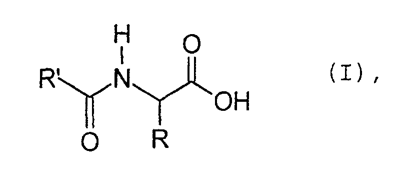 worin
R Wasserstoff, eine Carboxylgruppe, eine (C₁-C₁₂)-Alkylgruppe, die gesättigt, geradkettig, verzweigt oder zyklisch vorliegen kann, einen (C₂-C₁₂)-Alkenylrest, der ein- oder mehrfach ungesättigt, geradkettig, verzweigt oder zyklisch vorliegen kann, und eine (C₁-C₈)-Acyloxygruppe sowie einen (C₅-C₁₈)-Arylrest,
R' Wasserstoff, einen gesättigten, geradkettigen, verzweigten oder zyklischen (C₁-C₂₆)-Alkyl, einen ein- oder mehrfach ungesättigten, geradkettigen, verzweigten oder zyklischen (C₂-C₂₄)-Alkenylrest, einen (C₆-C₁₈)-Alykl- (C₅-C₁₈)-Arylrest oder einen ggf. mehrfach ungesättigten (C₂-C₁₀)-Alkenyl-(C₅-C₁₈)-Arylrest,
**dadurch gekennzeichnet,**
**daß** man ein Nitril der allgemeinen Formel II
R'CN (II),
in dem R' die oben angegebene Bedeutung besitzt,
mit einem Aldehyd der allgemeinen Formel III
R―CHO (III),
in dem R die oben angegebene Bedeutung besitzt, in Gegenwart von einer Säure, Kohlenmonoxid und einem Metallkatalysator zu Verbindungen der allgemeinen Formel I umsetzt.

2. Verfahren nach Anspruch 1,
**dadurch gekennzeichnet,**
**daß** man den Aldehyd in Form seiner Trimeren oder Oligomeren in die Reaktion einsetzt.

3. Verfahren nach Anspruch 1,
**dadurch gekennzeichnet,**
**daß** man die Umsetzung in Gegenwart einer Säure mit einem pka-Wert von < 4 durchführt.

4. Verfahren nach Anspruch 3,
**dadurch gekennzeichnet,**
**daß** man die Umsetzung in Gegenwart von Schwefelsäure oder einem Hydrogenhalogenid durchführt.

5. Verfahren nach Anspruch 4,
**dadurch gekennzeichnet,**
**daß** zusätzlich zur Säure Ameisensäure bei der Reaktion zugegen ist.

6. Verfahren nach Anspruch 1,
**dadurch gekennzeichnet,**
**daß** man als aktiven Metallkatalysator eine Palladium-(0)-Verbindung oder Cobalt-(0)-Verbindung benutzt.

7. Verfahren nach Anspruch 6,
**dadurch gekennzeichnet,**
**daß** man die Palladiumverbindung in einer Menge von 0,0001 bis 5 mol% bezogen auf das Nitril einsetzt.

8. Verfahren nach Anspruch 7,
**dadurch gekennzeichnet,**
**daß** man die Palladiumverbindung in einer Menge von 0,01 bis 2 mol% bezogen auf das Nitril einsetzt.

9. Verfahren nach einem der vorhergehenden Ansprüche,
**dadurch gekennzeichnet,**
**daß** man bei der Reaktion ein Halogenidsalz zusetzt.

10. Verfahren nach Anspruch 9,
**dadurch gekennzeichnet,**
**daß** man das Halogenidsalz in einem Konzentrationsbereich von 0,1 bis 100 mol% bezogen auf das Nitril einsetzt.

11. Verfahren nach einem der vorhergehenden Ansprüche,
**dadurch gekennzeichnet,**
**daß** man die Reaktion in dipolar aprotischen Lösungsmitteln oder Lösungsmittelgemischen ausführt.

12. Verfahren nach Anspruch 11,
**dadurch gekennzeichnet,**
**daß** man als Lösungsmittel N-Methylpyrrolidin, Dimethylformamid oder Acetonitril einsetzt.

13. Verfahren nach einem der vorhergehenden Ansprüche,
**dadurch gekennzeichnet,**
**daß** man bei einem Gasdruck von Kohlenmonoxid von 1 bis 250 bar arbeitet.

14. Verfahren nach Anspruch 13,
**dadurch gekennzeichnet,**
**daß** man bei einem CO-Druck von 10 - 150 bar arbeitet.

15. Verfahren nach einem der vorhergehenden Ansprüche,
**dadurch gekennzeichnet,**
**daß** man bei einer Temperatur von 0 bis 200 °C arbeitet.

16. Verfahren nach Anspruch 15,
**dadurch gekennzeichnet,**
**daß** man bei einer Temperatur von 50 bis 150 °C arbeitet.

17. Verfahren nach einem der vorhergehenden Ansprüchen,
**dadurch gekennzeichnet,**
**daß** man das Verfahren in einer Stufe ausführt.

18. Verfahren nach Anspruch 1,
**dadurch gekennzeichnet,**
**daß** man zur Herstellung der enantiomer angereicherten N-Acylaminosäuren chiral modifizierte Metallkatalysatoren verwendet.

## Claims

1. Process for the production of N-acyl amino acids of the general formula I 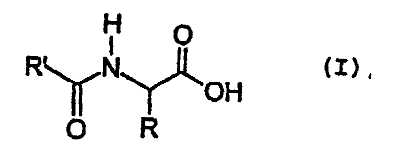 wherein
R hydrogen, a carboxyl group, a (C₁-C₁₂) alkyl group, which can be saturated, straight-chained, branched or cyclic, a (C₂-C₁₂) alkylene radical, which can be mono- or polyunsaturated, straight-chained, branched or cyclic, and a (C₁-C₈) acyloxy group and a (C₅-C₁₈) aryl radical,
R' hydrogen, a saturated, straight-chained, branched or cyclic (C₁-C₂₆) alkyl, a mono- or polyunsaturated, straight-chained, branched or cyclic (C₂-C₂₄) alkenyl radical, a (C₆-C₁₈) alkyl-(C₅-C₁₈) aryl radical or an optionally polyunsaturated (C₂-C₁₀) alkenyl-(C₅-C₁₈) aryl radical,
**characterised in that**
a nitrile of the general formula II
R'CN (II),
in which R' has the above meaning, is reacted with an aldehyde of the general formula III
R-CHO (III),
in which R has the above meaning,
in the presence of an acid, carbon monoxide and a metal catalyst to give compounds of the general formula I.

2. Process according to claim 1,
**characterised in that**
the aldehyde is fed into the reaction in the form of its trimers or oligomers.

3. Process according to claim 1,
**characterised in that**
the reaction is performed in the presence of an acid with a pKa value of < 4.

4. Process according to claim 3,
**characterised in that**
the reaction is performed in the presence of sulfuric acid or a hydrogen halide.

5. Process according to claim 4,
**characterised in that**
formic acid is present in the reaction in addition to the acid.

6. Process according to claim 1,
**characterised in that**
a palladium(0) compound or cobalt(0) compound is employed as the active metal catalyst.

7. Process according to claim 6,
**characterised in that**
the palladium compound is used in a quantity of 0.0001 to 5 mole %, based on the nitrile.

8. Process according to claim 7,
**characterised in that**
the palladium compound is used in a quantity of 0.01 to 2 mole %, based on the nitrile.

9. Process according to one of the above claims,
**characterised in that**
a halide salt is added during the reaction.

10. Process according to claim 9,
**characterised in that**
the halide salt is used in a concentration range of 0.1 to 100 mole %, based on the nitrile.

11. Process according to one of the above claims,
**characterised in that**
the reaction is carried out in dipolar aprotic solvents or solvent mixtures.

12. Process according to claim 11,
**characterised in that**
N-methylpyrrolidine, dimethylformamide or acetonitrile is used as the solvent.

13. Process according to one of the above claims,
**characterised in that**
work is carried out under a carbon monoxide gas pressure of 1 to 250 bar.

14. Process according to claim 13,
**characterised in that**
work is carried out under a CO pressure of 10 - 150 bar.

15. Process according to one of the above claims,
**characterised in that**
work is carried out at a temperature of 0 to 200°C.

16. Process according to claim 15,
**characterised in that**
work is carried out at a temperature of 50 to 150°C.

17. Process according to one of the above claims,
**characterised in that**
the process is carried out in one step.

18. Process according to claim 1,
**characterised in that**
chirally modified metal catalysts are used to produce the enantiomerically concentrated N-acyl amino acids.

## Revendications

1. Procédé de préparation d'acides N-acylaminés de formule générale I 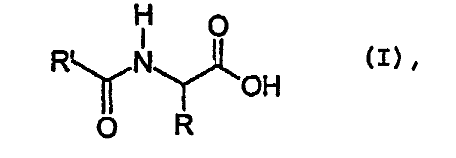 dans laquelle
R représente un hydrogène, un groupe carboxylique, un groupe alkyle en C₁ à C₁₂, qui peut se présenter sous forme saturée, à chaîne droite, ramifiée ou cyclique, un radical alcényle en C₂ à C₁₂, qui peut se présenter sous forme mono- ou polyinsaturée, à chaîne droite, ramifiée ou cyclique, et un groupe acyloxy en C₁ à C₈ ainsi qu'un radical aryle en C₅ à C₁₈,
R' représente un hydrogène, un radical alkyle en C₁ à C₂₆, saturé, à chaîne droite, ramifié ou cyclique, un radical alcényle en C₂ à C₂₄ mono- ou polyinsaturé, à chaîne droite, ramifié ou cyclique, un radical alkyle en C₆ à C₁₈-aryle en C₅ à C₁₈ ou un radical alcényle en C₂ à C₁₀-aryle en C₅ à C₁₈ éventuellement polyinsaturé,
**caractérisé en ce qu'**
on fait réagir un nitrile de formule générale II
R'CN (II)
dans laquelle R' possède la signification indiquée ci-dessus, avec un aldéhyde de formule générale III
R-CHO (III),
dans laquelle R possède la signification indiquée ci-dessus, en présence d'un acide, de monoxyde de carbone et d'un catalyseur métallique pour donner des composés de formule générale I désirée.

2. Procédé selon la revendication 1,
**caractérisé en ce qu'**
on utilise l'aldéhyde sous la forme de ses trimères ou oligomères dans la réaction.

3. Procédé selon la revendication 1,
**caractérisé en ce qu'**
on conduit la réaction en présence d'un acide avec une valeur de pKa inférieure à 4.

4. Procédé selon la revendication 3,
**caractérisé en ce qu'**
on conduit la réaction en présence d'acide sulfurique ou d'un acide halogénhydrique.

5. Procédé selon la revendication 4,
**caractérisé en ce qu'**
on trouve, outre l'acide, de l'acide formique dans la réaction.

6. Procédé selon la revendication 1,
**caractérisé en ce qu'**
on utilise comme catalyseur métallique actif un composé de palladium(0) ou de cobalt(0).

7. Procédé selon la revendication 6,
**caractérisé en ce qu'**
on utilise le composé de palladium en une quantité de 0,0001 à 5 % molaire par rapport au nitrile.

8. Procédé selon la revendication 7,
**caractérisé en ce qu'**
on utilise le composé de palladium en une quantité de 0,01 à 2 % molaire par rapport au nitrile.

9. Procédé selon l'une des revendications précédentes,
**caractérisé en ce qu'**
on ajoute dans la réaction un sel d'halogénure.

10. Procédé selon la revendication 9,
**caractérisé en ce qu'**
on utilise ces halogénures dans un intervalle de concentration allant de 0,1 à 100 % molaire par rapport au nitrile.

11. Procédé selon l'une des revendications précédentes,
**caractérisé en ce que**
l'on conduit la réaction dans les solvants ou mélanges de solvants aprotiques dipolaires.

12. Procédé selon la revendication 11,
**caractérisé en ce qu'**
on utilise comme solvant la N-méthyl pyrrolidine, le diméthyl formamide ou l'acétonitrile.

13. Procédé selon l'une des revendications précédentes,
**caractérisé en ce qu'**
on travaille à une pression de gaz de monoxyde de carbone de 1 à 250 bars.

14. Procédé selon la revendication 13,
**caractérisé en ce qu'**
on travaille à une pression de CO de 10 à 150 bars.

15. Procédé selon l'une des revendications précédentes,
**caractérisé en ce qu'**
on travaille à une température de 0 à 200°C.

16. Procédé selon la revendication 15,
**caractérisé en ce qu'**
on travaille à une température de 50 à 150°C.

17. Procédé selon l'une des revendications précédentes,
**caractérisé en ce qu'**
on effectue le procédé en une étape.

18. Procédé selon la revendication 1,
**caractérisé en ce que**
pour préparer des acides N-acylaminés enrichis en énantiomères, on utilise des catalyseurs métalliques à modification chirale.
